# EUROPÄISCHE PATENTSCHRIFT

(11) **EP 2 089 157 B1**
(45) Veröffentlichungstag und Bekanntmachung des Hinweises auf die Patenterteilung: **05.09.2012**
(21) Anmeldenummer: 07822192.6
(22) Anmeldetag: 05.11.2007
(51) Int. Cl.: B01J 31/16, B01J 20/00

(54) **MAGNESIUMBUTYLISOPHTHALAT ALS PORÖSES METALLORGANISCHES GERÜSTMATERIAL**
MAGNESIUM BUTYLISOPHTHALATE AS A POROUS METAL ORGANIC FRAMEWORK MATERIAL
ISOPHTALATE BUTYLIQUE DE MAGNÉSIUM EN GUISE DE MATÉRIAU D'OSSATURE ORGANOMÉTALLIQUE POREUX

(30) Priorität: 06.11.2006 EP 06123525
(43) Veröffentlichungstag der Anmeldung: 19.08.2009
(73) Patentinhaber: BASF SE, 67056 Ludwigshafen (DE)
(72) Erfinder: TRUKHAN, Natalia, 67063 Ludwigshafen (DE); MÜLLER, Ulrich, 67435 Neustadt (DE); SCHUBERT, Markus, 67063 Ludwigshafen (DE)
(86) Internationale Anmeldenummer: PCT/EP2007/061863
(87) Internationale Veröffentlichungsnummer: WO 2008/055862

(56) Entgegenhaltungen:
- WO-A-2005/049892
- PAN, LONG ET AL: "Zn(tbip) (H2tbip = 5-tert-Butyl Isophthalic Acid): A Highly Stable Guest-Free Microporous Metal Organic Framework with Unique Gas Separation Capability" JOURNAL OF THE AMERICAN CHEMICAL SOCIETY , 128(13), 4180-4181 CODEN: JACSAT; ISSN: 0002-7863, 14. März 2006 (2006-03-14), XP002464907
- SU ET AL: "Exceptionally stable, hollow, tubular matal-organic architectures: Synthesis, characterization, and solid-state transformation study" J. AM CHEM. SOC., Bd. 126, 2004, Seiten 3576-3586, XP002464908

## Beschreibung

Die vorliegende Erfindung betrifft ein poröses metallorganisches Gerüstmaterial, dessen Herstellung sowie Verwendung.

Poröse metallorganische Gerüstmaterialien sind im Stand der Technik bekannt. Sie zeichnen sich insbesondere durch ihre Porosität aus und sind vergleichbaren Anwendungen zuführbar, wie anorganische Zeolithe.

Metallorganische Gerüstmaterialien enthalten üblicherweise eine an ein Metallion koordinativ gebundene, mindestens zweizähnige organische Verbindung, die gemeinsam mit dem Metallion das Gerüst des metallorganischen Gerüstmaterials darstellt.

Die geeignete Wahl von Metall und/oder organischer Verbindung ermöglicht eine Optimierung für das gewünschte Anwendungsgebiet. Hierbei kann beispielsweise die Wahl der organischen Verbindung Einfluss auf die Porenverteilung nehmen. Darüber hinaus kann das Metall einen Beitrag bei Adsorptionsvorgängen liefern.

Es existiert also ein stetiger Bedarf, spezielle metallorganische Gerüstmaterialien bereitzustellen, die insbesondere außergewöhnliche Eigenschaften aufweisen, welche auf die Wahl des Metalls sowie der organischen Verbindung zurückzuführen sind.

Als ein interessantes Metall kann Magnesium genannt werden, da aufgrund starker Koordinationsbindungen von einem vergleichsweise engporigen Gerüstmaterial ausgegangen werden kann und weil Magnesium ein vergleichsweise physiologisch wie ökologisch verträgliches Metall darstellt.

M. Dinca et al., J. Am. Chem. Soc. 127 (2005), 9376 - 9377 beschreiben Magnesium-2,6-naphthalindicarboxylat als mikroporösen koordinativ aufgebauten Feststoff. Dieses Gerüstmaterial zeigt eine analoge Struktur zu dem entsprechenden Zink-basierten metallorganischen Gerüstmaterial. Die Autoren haben bei der Untersuchung des Materials festgestellt, dass dieses eine hohe Wasserstoffadsorptionsenthalpie sowie eine selektive Aufnahme von Wasserstoff oder Sauerstoff gegenüber Stickstoff oder Kohlenmonoxid aufweist.

In WO-A 2005/049892 wird die elektrochemische Herstellung von Magnesiumterephthalat in Gegenwart von Maleinsäurediethylester als poröses metallorganisches Gerüstmaterial beschrieben. Das so erhaltene Gerüstmaterial ist in Bezug auf seine Struktur ebenfalls mit dem entsprechenden Zink-basierten metallorganischen Gerüstmaterial vergleichbar.

Trotz der erfolgversprechenden Resultate des im Stand der Technik bekannten Magnesium-basierten metallorganischen Gerüstmaterials besteht nach wie vor ein Bedarf nach alternativen Gerüststrukturen, die durch geeignete Wahl von Metall und organischer Verbindung erzielt werden können.

Eine Aufgabe der vorliegenden Erfindung liegt somit darin, ein solches Magnesiumbasiertes metallorganisches Gerüstmaterial bereitzustellen.

Die Aufgabe wird gelöst durch ein poröses metallorganisches Gerüstmaterial, gebildet aus Mg²⁺-Ionen, an die koordinativ 5-tert.-Butylisophthalationen unter Ausbildung einer Gerüststruktur gebunden sind.

Es wurde nämlich überraschenderweise gefunden, dass das erfindungsgemäße Gerüstmaterial im Vergleich zum analogen Magnesiumisophthalat eine überraschend große spezifische Oberfläche aufweist und insbesondere für Trennungen von Gasen, die gasförmiges Wasser enthalten können, geeignet ist.

Das erfindungsgemäße Gerüstmaterial wird aus Mg²+ -Ionen und 5-tert.-Butylisophthalsäure (5-^{t}Butyl-1,3-benzoldicarbonsäure) bzw. deren anionischen Formen gebildet.

Das erfindungsgemäße metallorganische Gerüstmaterial kann pulverförmig beziehungsweise als Agglomerat vorliegen.

Das erfindungsgemäße poröse metallorganische Gerüstmaterial kann als solches in Pulverform verwendet werden oder es wird in einen Formkörper umgewandelt. Dementsprechend ist ein weiterer Aspekt der vorliegenden Erfindung, dass das erfindungsgemäße poröse metallorganische Gerüstmaterial als Teil eines Formkörpers vorliegt.

Die Herstellung von Formkörpern aus metallorganischen Gerüstmaterialien ist beispielsweise in WO-A 03/102000 beschrieben.

Bevorzugte Verfahren zur Herstellung von Formkörpern sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Absorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens
   einer Templatverbindung unter Erhalt eines Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Tablettieren zusammen mit mindestens einem Bindemittel und/oder anderem Hilfsstoff.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders bevorzugt werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl Viskositätserhöhende als auch Viskositätserniedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Halloysit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen.

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 500 °C, bevorzugt im Bereich von 50 bis 500 °C und besonders bevorzugt im Bereich von 100 bis 350 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Das erfindungsgemäße metallorganische Gerüstmaterial enthält Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm. Die Anwesenheit von Mikro- und/oder Mesoporen kann mit Hilfe von Sorptionsmessungen überprüft werden, wobei diese Messungen die Aufnahmekapazität der metallorganischen Gerüstmaterialien für Stickstoff bei 77 Kelvin gemäß DIN 66131 und/oder DIN 66134 bestimmt.

Vorzugsweise beträgt die spezifische Oberfläche - berechnet nach dem Langmuir-Modell (DIN 66131, 66134) für ein MOF in Pulverform bei mehr als 5 m²/g, mehr bevorzugt über 10 m²/g, mehr bevorzugt mehr als 50 m²/g, weiter mehr bevorzugt mehr als 100 m²/g, weiter mehr bevorzugt mehr als 200 m²/g und besonders bevorzugt mehr als 300 m²/g.

Formkörper aus metallorganischen Gerüstmaterialien können eine niedrigere spezifische Oberfläche besitzen; vorzugsweise jedoch mehr als 10 m²/g, mehr bevorzugt mehr als 50 m²/g, weiter mehr bevorzugt mehr als 100 m²/g, insbesondere mehr als 200 m²/g.

Vorzugsweise beträgt die Porengröße des erfindungsgemäßen metallorganischen Gerüstmaterials von 0,2 nm bis 30 nm, besonders bevorzugt liegt die Porengröße im Bereich von 0,3 nm bis 3 nm bezogen auf das kristalline Material.

In einem Formkörper des erfindungsgemäßen metallorganischen Gerüstmaterials treten jedoch auch größere Poren auf, deren Größenverteilung variieren kann. Vorzugsweise wird jedoch mehr als 50 % des gesamten Porenvolumens, insbesondere mehr als 75 %, von Poren mit einem Porendurchmesser von bis zu 1000 nm gebildet. Vorzugsweise wird jedoch ein Großteil des Porenvolumens von Poren aus zwei Durchmesserbereichen gebildet. Es ist daher weiter bevorzugt, wenn mehr als 25 % des gesamten Porenvolumens, insbesondere mehr als 50 % des gesamten Porenvolumens von Poren gebildet wird, die in einem Durchmesserbereich von 100 nm bis 800 nm liegen und wenn mehr als 15 % des gesamten Porenvolumens, insbesondere mehr als 25 % des gesamten Porenvolumens von Poren gebildet wird, die in einem Durchmesserbereich von bis zu 10 nm liegen. Die Porenverteilung kann mittels Quecksilber-Porosimetrie bestimmt werden.

Ein weiterer Gegenstand der vorliegenden Erfindung ist ein Verfahren zur Herstellung eines erfindungsgemäßen porösen metallorganischen Gerüstmaterials, den Schritt enthaltend
- Umsetzung einer Magnesiumverbindung mit 5-tert.-Butylisophthalsäure oder einem Salz davon.

Als organische Komponente des erfindungsgemäßen porösen metallorganischen Gerüstmaterials dient 5-tert.-Butylisophthalsäure, die mit einer Magnesiumverbindung umgesetzt werden kann. Ebenso ist es möglich, Derivate der 5-tert.-Butylisophthalsäure einzusetzen. So ist es zum Beispiel denkbar, dass 5-tert.-Butylisophthalsäure in Form ihres Salzes eingesetzt wird. Das Salz, bei dem 5-tert.-Butylisophthalsäure als vollständig oder teilweise deprotoniertes Anion vorliegt, kann ein beliebig geeignetes Kation aufweisen. Solche Kationen können beispielsweise ein- oder zweiwertige Metallionen sein. Beispiele hierfür sind insbesondere Natrium- und Kaliumsalze. Ebenso sind Kationen von Ammoniumverbindungen einsetzbar. Hierbei sind insbesondere Ammonium selbst sowie Alkylammoniumkationen zu nennen.

Die Magnesiumverbindung kann durch anodische Oxidation von metallischem Magnesium erzeugt werden. In einem solchen Fall wird das erfindungsgemäße poröse metallorganische Gerüstmaterial auf elektrochemischem Wege hergestellt. Verfahren zur elektrochemischen Herstellung von porösen metallorganischen Gerüstmaterialien sind in WO-A 2005/049892 beschrieben. Auch das erfindungsgemäße poröse metallorganische Gerüstmaterial kann auf diesem Wege hergestellt werden.

Bei der elektrochemischen Herstellung des erfindungsgemäßen porösen metallorganischen Gerüstmaterials ist bevorzugt, dass die kathodische Wiederabscheidung des Magnesiumions durch mindestens eine der folgenden Maßnahmen zumindest teilweise verhindert wird:
(i) Verwendung eines Elektrolyten, der die kathodische Bildung von Wasserstoff begünstigt;
(ii) Zusatz mindestens einer Verbindung, die zu einer kathodischen Depolarisation führt;
(iii) Einsatz einer Kathode mit einer geeigneten Wasserstoffüberspannung.

Das Verfahren kann in einer ungeteilten Elektrolysezelle durchgeführt werden. Speziell geeignete Zellen sind Spaltzellen oder Plattenstapelzellen. Diese können bipolar geschaltet sein. Als Reaktionsmedium eignet sich beispielsweise Methanol, Ethanol, Dimethylformamid, Diethylformamid oder ein Gemisch aus zwei oder mehr dieser Lösemittel.

In dem Reaktionsmedium kann/können weiterhin ein Leitsalz oder mehrere Leitsalze vorhanden sein. Hierbei kann das Leitsalz als Kationkomponente ein quartäres Ammonium und als Anionkomponente ein Alkoxysulfat aufweisen. Der Gesamtfeststoffgehalt sollte im Bereich von größer oder gleich 0,5 Gew.-% liegen.

Die Umsetzung in dem erfindungsgemäßen Verfahren zur Herstellung des erfindungsgemäßen metallorganischen Gerüstmaterials kann auch auf klassischem Wege erfolgen. Hierbei ist die Magnesiumverbindung typischerweise ein Magnesiumsalz.

Das Magnesiumsalz kann in Form eines Alkoholats, Acetonats, Halegonids, Sulfits, als Salz einer organischen oder anorganischen, Sauerstoff enthaltenden Säure oder einer Mischung davon vorliegen.

Ein Alkoholat ist beispielsweise ein Methanolat, Ethanolat, n-Propanolat, i-Propanolat, n-Butanolat, i-Butanolat, t-Butanolat oder Phenolat.

Ein Acetonat ist beispielsweise Acetylacetonat.

Ein Halogenid ist beispielsweise Chlorid, Bromid oder Iodid.

Eine organische, Sauerstoff enthaltende Säure ist beispielsweise Ameisensäure, Essigsäure, Propionsäure oder andere Alkylmonocarbonsäuren.

Eine anorganische, Sauerstoff enthaltende Säure ist beispielsweise Schwefelsäure, schweflige Säure, Phosphorsäure oder Salpetersäure.

Hierbei tritt das Magnesium als Mg²⁺-Kation auf.

Weiter bevorzugte Magnesiumverbindungen sind anorganische Magnesiumsalze wie Magnesiumchlorid, Magnesiumbromid, Magnesiumhydrogensulfat, Magnesiumdihydrogenphosphat, Magnesiummonohydrogenphosphat, Magnesiumnitrat.

Die Magnesiumverbindung kann gegebenenfalls Hydratwasser aufweisen.

Die Umsetzung in dem erfindungsgemäßen Verfahren zur Herstellung des erfindungsgemäßen porösen metallorganischen Gerüstmaterials kann in wässrigem Medium erfolgen. Hierbei können Hydrothermalbedingungen oder allgemein Solvothermalbedingungen eingesetzt werden. Unter dem Begriff "thermal" ist im Rahmen der vorliegenden Erfindung ein Herstellverfahren zu verstehen, bei dem die Umsetzung zum erfindungsgemäßen porösen metallorganischen Gerüstmaterial in einem Druckbehälter derart durchgeführt wird, dass dieser während der Umsetzung verschlossen ist und erhöhte Temperatur angelegt wird, so dass aufgrund des Dampfdruckes von vorhandenem Lösemittel sich ein Druck innerhalb des Reaktionsmediums im Druckbehälter aufbaut.

Vorzugsweise erfolgt die Umsetzung jedoch nicht in wässrigem Medium und ebenso nicht unter Solvothermalbedingungen.

Die Umsetzung in dem erfindungsgemäßen Verfahren erfolgt vorzugsweise in Gegenwart eines nicht-wässrigen Lösemittels.

Die Umsetzung erfolgt vorzugsweise bei einem Druck von höchstens 2 bar (absolut). Vorzugsweise beträgt der Druck jedoch höchstens 1230 mbar (absolut). Insbesondere bevorzugt findet die Umsetzung bei Atmosphärendruck statt. Hierbei kann es jedoch apparativbedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlichen vorliegenden Atmosphärendruck ± 150 mbar ergibt.

Die Umsetzung kann bei Raumtemperatur durchgeführt werden. Vorzugsweise findet diese jedoch bei Temperaturen oberhalb der Raumtemperatur statt. Vorzugsweise beträgt die Temperatur mehr als 100 °C. Weiterhin bevorzugt beträgt die Temperatur höchstens 180 °C und mehr bevorzugt höchstens 150 °C.

Typischerweise werden die oben beschriebenen metallorganischen Gerüstmaterialien in Wasser als Lösemittel unter Zusatz einer weiteren Base durchgeführt. Dies dient insbesondere dazu, dass bei Einsatz einer mehrbasigen Carbonsäure als mindestens zweizähniger organischer Verbindung diese leicht löslich in Wasser ist. Durch die bevorzugte Verwendung des nicht-wässrigen organischen Lösemittels ist es nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Es ist weiterhin vorteilhaft, dass die Umsetzung unter Rühren stattfinden kann, was auch bei einem Scale-up vorteilhaft ist.

Das nicht-wässrige organische Lösemittel ist vorzugsweise ein C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetra hydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind DMF, DEF und NMP. Besonders bevorzugt ist DMF.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Weiterhin bevorzugt schließt sich an den Verfahrensschritt der Umsetzung der mindestens einen Metallverbindung mit der mindestens einen mindestens zweizähnigen organischen Verbindung ein Calcinierungsschritt an. Die hierbei eingestellte Temperatur beträgt typischerweise mehr als 250 °C, bevorzugt 300 bis 400 °C.

Aufgrund des Calcinierungsschrittes kann die in den Poren befindliche mindestens zweizähnige organische Verbindung entfernt werden.

Ergänzend oder alternativ hierzu kann die Entfernung der mindestens zweizähnigen organischen Verbindung (Ligand) aus den Poren des porösen metallorganischen Gerüstmaterials durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt. Diese schonende Methode ist insbesondere geeignet, wenn es sich bei dem Liganden um eine hochsiedende Verbindung handelt.

Die Behandlung erfolgt vorzugsweise mindestens 30 Minuten und kann typischerweise bis zu 2 Tagen durchgeführt werden. Dies kann bei Raumtemperatur oder erhöhter Temperatur geschehen. Vorzugsweise erfolgt dies unter erhöhter Temperatur, beispielsweise bei mindestens 40 °C, bevorzugt 60 °C. Weiterhin bevorzugt erfolgt die Extraktion bei der Siedetemperatur des eingesetzten Lösemittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen, eingesetzt werden.

Als geeignete Lösemittel können die oben genannten verwendet werden, also beispielsweise C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon oder Mischungen davon.

Bevorzugt sind Methanol, Ethanol, Propanol, Aceton, MEK und Mischungen davon.

Ein ganz besonders bevorzugtes Extraktionslösemittel ist Methanol.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Insbesondere ist es bei der "Extraktion" nicht unbedingt erforderlich aber bevorzugt, dass das Lösemittel wasserfrei ist.

Ein weiterer Gegenstand der vorliegenden Erfindung ist die Verwendung eines erfindungsgemäßen porösen metallorganischen Gerüstmaterials zur Aufnahme mindestens eines Stoffes, zu dessen Speicherung, Abtrennung, kontrollierten Abgabe oder chemischen Umsetzung.

Vorzugsweise handelt es sich bei dem mindestens einen Stoff um ein Gas oder Gasgemisch, wobei das Gas oder Gasgemisch vorzugsweise gasförmiges Wasser enthält.

Hierdurch ist insbesondere eine Abtrennung von Gasen oder Gasgemischen in Gegenwart von Wasser möglich, ohne dass das Wasser die Trennung dahingehend stört, dass dieses anstelle des Gases oder Gasgemisches abgetrennt wird.

Verfahren zur Speicherung mit Hilfe von metallorganischen Gerüstmaterialien im Allgemeinen sind in WO-A 2005/003622, WO-A 2003/064030, WO-A 2005/049484 sowie in WO-A 2006/089908 und DE-A 10 2005 012 087 beschrieben. Die dort beschriebenen Verfahren können auch für das erfindungsgemäße metallorganische Gerüstmaterial eingesetzt werden.

Verfahren zur Trennung beziehungsweise Reinigung mit Hilfe von metallorganischen Gerüstmaterialien im Allgemeinen sind in EP 1 674 555 sowie DE-A 10 2005 000938 und DE-A 10 2005 022 844 beschrieben. Die dort beschriebenen Verfahren können auch für das erfindungsgemäße metallorganische Gerüstmaterial eingesetzt werden.

Sofern das erfindungsgemäße poröse metallorganische Gerüstmaterial zur Speicherung eingesetzt wird, erfolgt dies vorzugsweise in einem Temperaturbereich von -200 °C bis +80 °C. Mehr bevorzugt ist ein Temperaturbereich von -40 °C bis +80 °C.

Bei dem mindestens einen Stoff kann es sich um ein Gas oder eine Flüssigkeit handeln. Vorzugsweise handelt es sich bei dem Stoff um ein Gas.

Im Rahmen der vorliegenden Erfindung werden vereinfachend die Begriffe "Gas" und "Flüssigkeit" verwendet; wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Erdgas, Stadtgas, Kohlenwasserstoffe, insbesondere Methan, Ethan, Ethin, Acetylen, Propan, n-Butan sowie i-Butan, Kohlenmonoxid, Kohlendioxid, Stickoxide, Sauerstoff, Schwefeloxide, Halogene, Halogenide Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon.

Bei dem mindestens einen Stoff kann es sich jedoch auch um eine Flüssigkeit handeln. Beispiele für eine solche Flüssigkeit sind Desinfektionsmittel, anorganische oder organische Lösemittel, Treibstoffe - insbesondere Benzin oder Diesel -, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyrridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder Iod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskateller Salbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butyl- alpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300 °C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20 °C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20 °C) auf, mehr bevorzugt einen Dampfdruck von mehr als 0,05 kPa (20 °C) auf. Besonders bevorzugt weisen die Geruchsstoffe einen Dampfdruck von mehr als 0,1 kPa (20 °C) auf.

Darüber hinaus hat es sich als vorteilhaft erwiesen, dass das erfindungsgemäße poröse metallorganische Gerüstmaterial dafür verwendet werden kann, ein Magnesiumoxid herzustellen. Dabei wird das erfindungsgemäße metallorganische Gerüstmaterial über dessen vollständige Zersetzungstemperatur erhitzt.

Das Erhitzen kann durch für den Fachmann bekannte Methoden erfolgen. Typischerweise erfolgt das Erhitzen in einem dazu geeigneten Ofen, wie beispielsweise einem Muffelofen. Bei Verwendung eines Ofens ist es weiterhin zweckdienlich, dass Möglichkeiten vorhanden sind, das Erhitzen in Gegenwart einer geeigneten Atmosphäre durchführen zu können. Hierzu kann entsprechend eine Zuführung für ein entsprechendes Gas oder Gasgemisch derart im oder am Ofen angebracht sein, damit der das poröse metallorganische Gerüstmaterial enthaltende Ofenraum mit dem entsprechenden Gas oder Gasgemisch geflutet werden kann.

Das poröse metallorganische Gerüstmaterial wird soweit erhitzt, wie es erforderlich ist, um das metallorganische Gerüstmaterial in das entsprechende Metalloxid umzuwandeln. Hierbei wird daher über die vollständige Zersetzungstemperatur des metallorganischen Gerüstmaterials erhitzt.

Im Rahmen der vorliegenden Erfindung ist unter "vollständige Zersetzungstemperatur" diejenige Temperatur zu verstehen, bei der das poröse metallorganische Gerüstmaterial beginnt, sich in das entsprechende Metalloxid umzuwandeln. Es ist jedoch ebenfalls möglich, dass das metallorganische Gerüstmaterial über Zwischenstufen zum Metalloxid umgewandelt wird. Beispielsweise könnte vor Bildung des Metalloxids ein Carbonat gebildet worden sein. In einem solchen Fall ist unter der "vollständigen Zersetzungstemperatur" diejenige Temperatur zu verstehen, die erforderlich ist, die jeweils letzte Zwischenstufe zum Metalloxid zu überführen.

Die Bestimmung der vollständigen Zersetzungstemperatur kann anhand der vom Fachmann bekannten Methoden durchgeführt werden. Beispielsweise kann durch Thermogravimetrie diese Temperatur bestimmt werden, wobei durch begleitende Analytik ebenfalls der Nachweis der Bildung des entsprechenden Metalloxids geführt werden kann.

Die vollständige Zersetzungstemperatur, die erforderlich ist, aus dem porösen metallorganischen Gerüstmaterial das entsprechende Metalloxid herzustellen, liegt typischerweise im Bereich von 250 °C bis 1000 °C. Weiterhin bevorzugt liegt die vollständige Zersetzungstemperatur in einem Bereich von 350 °C bis 800 °C. Insbesondere bevorzugt liegt die vollständige Zersetzungstemperatur im Bereich von 450 °C bis 650 °C.

Daher findet das Erhitzen des porösen metallorganischen Gerüstmaterials in Gegenwart einer oxidierenden Atmosphäre mit einem Sauerstoff liefernden Bestandteil statt. Hierdurch kann gewährleistet werden, dass für die Umwandlung des porösen metallorganischen Gerüstmaterials in das entsprechende Metalloxid ausreichend Sauerstoff zur Verfügung steht. Dies kann insbesondere auch dazu beitragen, dass die oben erwähnten Zwischenstufen "übersprungen" werden. Solche oxidierenden Atmosphären können durch entsprechend Sauerstoff liefernde Gase oder Gasgemische erhalten werden. Als einfachstes und bevorzugtes Gasgemisch ist hierbei Luft zu nennen, das normalerweise einen ausreichend hohen Anteil an molekularem Sauerstoff enthält. Gegebenenfalls kann die Luft mit weiterem Sauerstoff angereichert eingesetzt werden. Schließlich ist es selbstverständlich ebenso möglich, dass reiner Sauerstoff als oxidierende Atmosphäre verwendet wird. Darüber hinaus können auch andere Gase oder Gasgemische verwendet werden, die beispielsweise mit molekularem Sauerstoff angereichert sind. Hierbei wären insbesondere Inertgase bevorzugt. So können geeignete Gasgemische zur Erzeugung einer oxidierenden Atmosphäre bei Erhitzen des porösen metallorganischen Gerüstmaterials Helium, Argon, Stickstoff oder Gemische hiervon jeweils mit Sauerstoff angereichert eingesetzt werden.

Das erfindungsgemäße poröse metallorganische Gerüstmaterial kann einer oxidierenden Atmosphäre derart ausgesetzt sein, dass während des Erhitzens die Atmosphäre nicht verändert wird. Das das poröse metallorganische Gerüstmaterial umgebende Gas oder Gasgemisch wird somit nicht ausgetauscht, so dass der Sauerstoff liefernde Bestandteil der Atmosphäre während des Erhitzens abnimmt.

Darüber hinaus ist es möglich, die Atmosphäre während des Erhitzens in Bezug auf deren Sauerstoff liefernden Bestandteil durch Nachführen zumindest dieses Bestandteils in etwa konstant zu halten.

Bevorzugt ist jedoch, dass der Sauerstoff liefernde Bestandteil während des Erhitzens erhöht wird. Dies kann beispielsweise dadurch erfolgen, dass die Atmosphäre durch ein Gas oder Gasgemisch mit höherem Anteil an Sauerstoff lieferndem Bestandteil ausgetauscht wird. Dies kann insbesondere derart erfolgen, dass der Atmosphäre nach dem Beginn des Erhitzens Sauerstoff zugeführt wird, bis schließlich eine reine Sauerstoffatmosphäre vorliegt. Die Erhöhung kann schrittweise oder kontinuierlich erfolgen.

Beispiele, bei der eine chemische Umsetzung in Gegenwart des erfindungsgemäßen metallorganischen Gerüstmaterials stattfinden kann, stellen die Alkoxylierung von Monoolen sowie Polyolen dar. Die Durchführung solcher Alkoxylierungen sind WO-A 03/035717 sowie WO-A 2005/03069 beschrieben. Ebenso kann das erfindungsgemäße poröse metallorganische Gerüstmaterial zur Epoxidierung sowie Herstellung von Polyalkylencarbonaten und Wasserstoffperoxid eingesetzt werden. Solche Reaktionen sind in WO-A 03/101975, WO-A 2004/037895 sowie US-A 2004/081611 beschrieben.

### Beispiel 1

Eine Mischung von 9,5 g Magnesiumnitrathexahydrat, 2,78 g 5-tert.-Butylisophthalsäure und 283 g Diethylformamid (DEF) wird in einem 500 ml Kolben unter N₂-Atmosphäre für 24 h bei 130 °C gerührt. Danach wird die Mischung auf Raumtemperatur abgekühlt und das ausfallende Produkt abfiltriert, vier mal mit je 50 ml Aceton gewaschen und anschließend 2 Tage in einer Waschflasche mit Fritte mittels N₂ trockengeblasen.

Es ergibt sich ein getrocknetes Gerüstmaterial von 2,60 g.

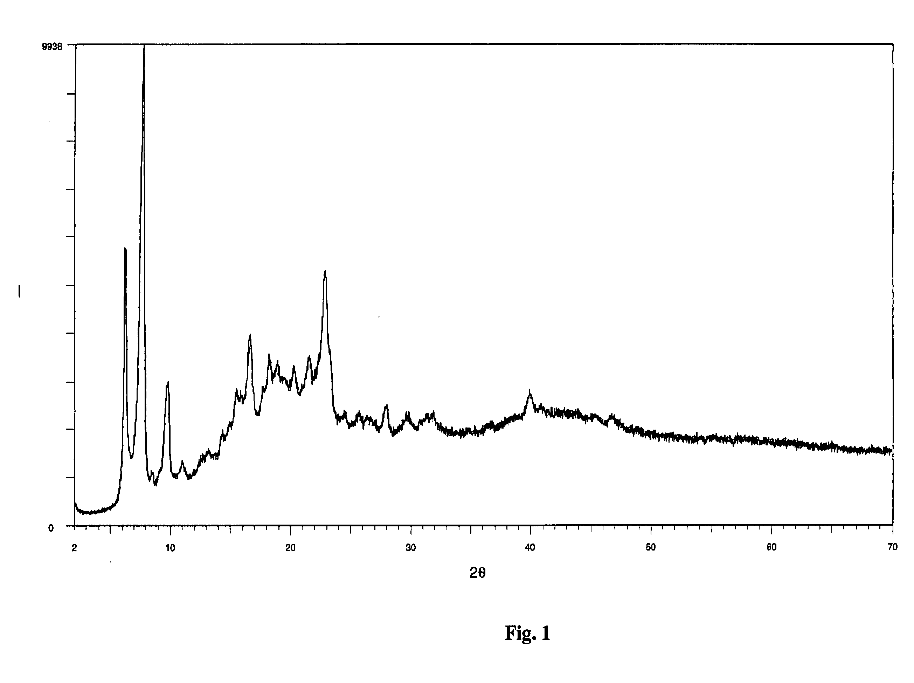

Fig. 1 zeigt das zugehörige Röntgendiffraktogramm (XRD), wobei I die Intensität (Lin(Counts)) angibt und 2Θ die 2-Theta-Skala beschreibt.

Die spezifische Oberfläche nach Langmuir ergibt einen Wert von 326 m²/g. Eine thermische Zersetzung erfolgt bei ca. 470 °C.

### Vergleichsbeispiel 2

Eine Mischung von 11,0 g Magnesiumnitrathexahydrat, 5,00 g 1,3-Benzoldicarbonsäure (Isophthalsäure) und Diethylformamid (DEF) wird in einem 200 ml Stahlautoklaven mit Tef-Ion-Innenbeschichtung für 24 h bei 130 °C geführt. Danach wird die Mischung auf Raumtemperatur abgekühlt und das ausfallende Produkt abfiltriert, mit N,N-Dimethylformamid (2 x 30 ml) und Chloroform (2 x 30 ml) gewaschen und anschließend an der Luft getrocknet.

Es ergibt sich ein getrocknetes Gerüstmaterial von 7,80 g.

Es konnte keine spezifische Oberfläche nach Langmuir bestimmt werden.

### Beispiel 3

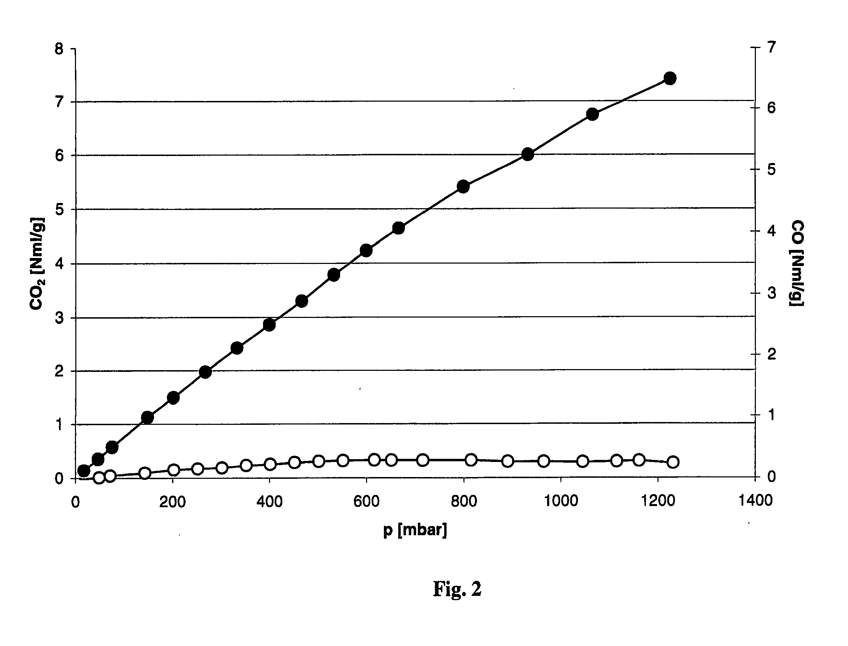

Fig. 2 zeigt die Adsorptionsisotherme des Gerüstmaterials aus Beispiel 1 für CO₂ und CO bei 313K. Die obere Kurve repräsentiert CO₂, die untere CO. Die Kurven demonstrieren, dass ein CO/CO₂-Trennung möglich ist.

## Patentansprüche

1. Poröses metallorganisches Gerüstmaterial gebildet aus Mg²⁺-Ionen, an die koordinativ 5-tert.-Butylisophthalationen unter Ausbildung einer Gerüststruktur gebunden sind.

2. Gerüstmaterial nach Anspruch 1, **dadurch gekennzeichnet, dass** dieses als Teil eines Formkörpers vorliegt.

3. Verfahren zur Herstellung eines metallorganischen Gerüstmaterials nach Anspruch 1 oder 2, den Schritt enthaltend
- Umsetzung einer Magnesiumverbindung mit 5-tert.-Butylisophthalsäure oder einem Salz davon.

4. Verfahren nach Anspruch 3, **dadurch gekennzeichnet, dass** die Magnesiumverbindung durch anodische Oxidation von metallischem Magnesium erzeugt wird.

5. Verfahren nach Anspruch 3, **dadurch gekennzeichnet, dass** die Magnesiumverbindung ein Magnesiumsalz ist.

6. Verwendung eines metallorganischen Gerüstmaterials nach Anspruch 1 oder 2 zur Aufnahme mindestens eines Stoffes zu dessen Speicherung, Abtrennung oder kontrollierten Abgabe.

7. Verwendung nach Anspruch 6, **dadurch gekennzeichnet, dass** der Stoff ein Gas oder Gasgemisch ist.

8. Verwendung nach Anspruch 7, **dadurch gekennzeichnet, dass** das Gas oder Gasgemisch gasförmiges Wasser enthält.

## Claims

1. A porous metal organic framework formed by Mg²⁺ ions to which 5-tert-butylisophthalate ions are coordinated to form a framework structure.

2. The framework according to claim 1 present as part of a shaped body.

3. A process for preparing a metal organic framework according to claim 1 or 2, which comprises the step
- reaction of a magnesium compound with 5-tert-butylisophthalic acid or a salt thereof.

4. The process according to claim 3, wherein the magnesium compound is produced by anodic oxidation of metallic magnesium.

5. The process according to claim 3, wherein the magnesium compound is a magnesium salt.

6. The use of a metal organic framework according to claim 1 or 2 for the uptake of at least one substance for the purposes of its storage, separation or controlled release.

7. The use according to claim 6, wherein the substance is a gas or gas mixture.

8. The use according to claim 7, wherein the gas or gas mixture comprises gaseous water.

## Revendications

1. Matériau d'ossature organométallique poreux à base d'ions Mg²⁺, auxquels sont liés par coordination des ions 5-tert-butylisophtalate avec formation d'une structure d'ossature.

2. Matériau d'ossature selon la revendication 1, **caractérisée en ce que** celui-ci est présent en tant que partie d'un corps moulé.

3. Procédé pour la production d'un matériau d'ossature organométallique selon la revendication 1 ou 2, comportant l'étape
- mise en réaction d'un composé de magnésium avec de l'acide 5-tert-butylisophtalique ou un sel de celui-ci.

4. Procédé selon la revendication 3, **caractérisé en ce que** le composé de magnésium est produit par oxydation anodique de magnésium métallique.

5. Procédé selon la revendication 3, **caractérisé en ce que** le composé de magnésium est un sel de magnésium.

6. Utilisation d'un matériau d'ossature organométallique selon la revendication 1 ou 2, pour la capture d'au moins une substance en vue de son emmagasinage, de sa séparation ou de sa libération programmée.

7. Utilisation selon la revendication 6, **caractérisée en ce que** la substance est un gaz ou mélange de gaz.

8. Utilisation selon la revendication 7, **caractérisée en ce que** le gaz ou mélange de gaz contient de l'eau sous forme de gaz.
